# DEMANDE DE BREVET EUROPEEN

(11) **EP 0 646 808 A1**
(43) Date de publication de la demande: **05.04.1995**
(21) Numéro de dépôt: 94402178.1
(22) Date de dépôt: 29.09.1994
(51) Int. Cl.: G01R 33/60, A61K 49/00

(54) **Procédé de contrôle de la teneur en monoxyde d'azote d'un milieu par résonance paramagnétique électronique en utilisant une phtalocyanine de lithium**

(30) Priorité: 01.10.1993 FR 9311713
(71) Demandeur: COMMISSARIAT A L'ENERGIE ATOMIQUE, F-75015 Paris Cédex 15 (FR)
(72) Inventeur: Moussavi, Mehdi, F-38120 Saint Egreve (FR); Duret, Denis, F-38100 Grenoble (FR)
(74) Mandataire: Des Termes, Monique

(57) **Abrégé**

L'invention concerne un procédé de contrôle de la teneur en monoxyde d'azote d'un milieu liquide et/ou gazeux.

Ce procédé consiste à mesurer le signal RPE de cristaux de phtalocyanine de lithium radicalaire en contact avec le milieu à contrôler. On peut ainsi déterminer des concentrations en NO de 0 à 16µmol/l en déterminant la largeur de raie dans un spectromètre de résonance paramagnétique électronique.

## Description

La présente invention a pour objet un procédé de contrôle de la teneur en monoxyde d'azote (NO) d'un milieu par résonance paramagnétique électronique.

Elle s'applique en particulier au dosage ou à la détection du monoxyde d'azote sous forme gazeuse ou dissoute dans un milieu liquide tel que l'eau, les liquides biologiques comme le sang et les milieux de culture de cellules comme le milieu KREBS.

L'intérêt pour le dosage du NO dans l'organisme se manifeste de plus en plus depuis les cinq dernières années au fur et à mesure des découvertes des propriétés de cette molécule. Ces découvertes ont en effet transformé la réputation de poison de NO en réputation de composé jouant un rôle fondamental pour l'organisme humain. NO a même été élu molécule de l'année 1992 dans la revue Science, vol. 258, 18 décembre 1992, page 1862-1865 où E. Culotta et D. Koshland décrivent un certain nombre des propriétés du NO, notamment son rôle de vasodilatateur.

Il est donc d'un grand intérêt de disposer de procédés de contrôle de la teneur en monoxyde d'azote (dosage ou détection) dans des milieux biologiques. Récemment, on a trouvé que le dosage du NO pouvait être effectué par la technique de résonance paramagnétique électronique (RPE). Cette technique a été décrite par Clarkson et al dans une communication faite à la onzième conférence de la Society of Magnetic Resonance in Medicine (SMRM) qui s'est tenue à Berlin en août 1992 : "Proceedings of the 11th Conference of SMRM Berlin, August 1992, p. 4108". Dans cette technique, on utilise un traceur radicalaire constitué par la fusinite dont les caractéristiques RPE telles que la largeur de raie, varient en fonction de la teneur en NO du milieu dans lequel se trouve ce traceur. On peut ainsi mesurer la concentration du NO intracellulaire en mettant en incubation des cellules en présence de fusinite et en examinant une suspension de ces cellules dans un liquide biologique désoxygéné, au moyen d'un spectromètre à résonance paramagnétique électronique. A partir de la largeur de raie RPE obtenue, on vérifie la présence de NO dans le milieu. L'élargissement de la raie RPE de la fusinite provient de l'interaction magnétique entre le traceur radicalaire (la fusinite) et la substance paramagnétique à contrôler (NO).

Toutefois, l'utilisation de fusinite pour contrôler la teneur en NO d'un milieu par RPE pose certains problèmes. En effet, la sensibilité n'est pas satisfaisante car la largeur de raie de la substance radicalaire est de 1 gauss environ en l'absence de NO et de 3 gauss en présence d'une quantité de NO qui n'est pas précisée. Par ailleurs, les auteurs ne donnent aucune courbe d'étalonnage et aucun chiffre pour la résolution.

D'autre part, la technique décrite par ces auteurs suppose que l'on désoxygène au préalable le milieu dans lequel on veut mesurer le NO. En effet, la substance paramagnétique utilisée ne fait pas la différence entre O₂ et NO qui sont tous deux des substances paramagnétiques. De ce fait, il est impossible de mesurer le taux de NO dans l'organisme ou même dans des cellules in vitro dans des conditions réelles de la vie qui supposent la présence d'oxygène.

La présente invention a précisément pour objet un procédé de contrôle de la teneur en NO d'un milieu par la technique RPE, qui utilise comme traceur radicalaire une substance ayant une meilleure sensibilité que la fusinite et qui peut être de plus mis en oeuvre de façon à mesurer sélectivement NO en présence d'oxygène.

Selon l'invention, le procédé de détection ou de dosage de monoxyde d'azote NO dans un milieu gazeux et/ou liquide, consiste :
a) à mettre en contact avec ledit milieu une phtalocyanine de lithium radicalaire de formule : 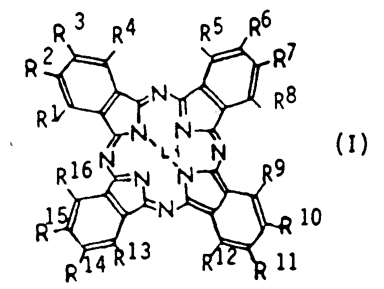 dans laquelle R¹, R⁴, R⁵, R⁸, R⁹, R¹², R¹³ et R¹⁶ représentent un atome d'hydrogène, et R², R³, R⁶, R⁷, R¹⁰, R¹¹, R¹⁴ et R¹⁵ qui peuvent être identiques ou différents, représentent un atome d'hydrogène, le groupe méthyle ou le groupe méthoxy, et
b) à examiner le signal de résonance paramagnétique électronique (RPE) de la phatlocyanine de lithium radicalaire en contact avec ledit milieu.

Dans ce procédé, l'utilisation d'une phtalocyanine de lithium radicalaire répondant à la formule (I) précitée permet d'obtenir une sensibilité élevée pour le dosage de NO dans la gamme de concentrations allant de 0 à 10µmol/l, qui constitue une gamme très intéressante car elle correspond aux concentrations que l'on rencontre habituellement dans les milieux biologiques.

Par ailleurs, on obtient dans cette gamme, une limite de résolution élevée puisqu'elle est de 10⁻⁸mol/l dans le cas de la phtalocyanine de lithium de formule (I) avec R¹ à R¹⁶ représentant H, et de 10⁻⁹mol/l dans le cas de la phtalocyanine de lithium de formule (I) avec R¹, R⁴, R⁵, R⁸, R⁹, R¹², R¹³ et R¹⁶ représentant H et R², R³, R⁶, R⁷, R¹⁰, R¹¹, R¹⁴ et R¹⁵ représentant le groupe méthoxy.

Les phtalocyanines de lithium radicalaire utilisées dans le procédé de l'invention peuvent être sous différentes formes cristallines. Ainsi, on peut utiliser les phtalocyanines de lithium décrites dans FR-A- 2 664 607 qui présentent une structure cristalline tétragonale appartenant au groupe spatial P4/mcc, et les phtalocyanines de lithium décrites dans FR-A- 2 664 705 qui sont généralement sous la forme de cristaux de structure tétragonale appartenant au groupe spatial P4/nnc.

Comme il est décrit dans les documents FR-A- 2 664 607 et 2 664 705, ces phtalocyanines de lithium sont également sensibles à l'oxygène. Toutefois, comme on le verra plus loin, lorsque le milieu à contrôler contient également de l'oxygène, on peut néanmoins utiliser ces phtalocyanines de lithium radicalaires pour le dosage de monoxyde d'azote en prévoyant de réaliser de plus un traitement du milieu à contrôler au moyen d'un agent complexant capable de complexer l'oxygène plus rapidement que le monoxyde d'azote.

Le procédé de l'invention peut être mis en oeuvre de différentes façons selon la nature du milieu à contrôler et le contrôle que l'on veut effectuer.

Ainsi, selon un premier mode de mise en oeuvre de ce procédé adapté au contrôle en continu de la teneur en NO d'un milieu gazeux et/ou liquide, on fait circuler en continu le milieu dans une conduite contenant des cristaux de phtalocyanine de lithium radicalaire et on examine en continu le signal RPE desdits cristaux.

Dans ce mode de mise en oeuvre du procédé, les cristaux peuvent être retenus dans la conduite entre deux pastilles poreuses, perméables au milieu à contrôler.

Ce premier mode de mise en oeuvre du procédé permet ainsi de détecter toute variation de concentration en NO d'un gaz et/ou d'un liquide ayant une concentration connue en NO, ce qui peut être intéressant dans le cas de solutions biologiques titrées ayant de faibles concentrations en NO.

Selon un second mode de mise en oeuvre du procédé de l'invention, destiné au dosage des teneurs en NO d'un milieu liquide et d'un milieux gazeux, on introduit lesdits milieux dans un récipient fermé comportant un tube latéral étanche à l'intérieur duquel sont retenus des cristaux de phtalocyanine de lithium radicalaire, ledit tube étant en communication avec le récipient et débouchant dans celui-ci à un niveau tel que, dans une première position du récipient, ce dernier contienne le milieu liquide sans qu'il vienne au contact desdits cristaux, on effectue dans cette première position une première mesure du signal RPE des cristaux qui sont en contact uniquement avec le milieu gazeux, puis on amène le récipient dans une seconde position dans laquelle le milieu liquide pénnètre dans le tube latéral pour être en contact avec lesdits cristaux et on effectue une seconde mesure de signal RPE desdits cristaux qui sont en contact uniquement avec le milieu liquide.

Selon un troisième mode de mise en oeuvre du procédé, destiné au dosage ou à la détection de monoxyde d'azote dans un milieu contenant également de l'oxygène, on met le milieu en contact successivement ou simultanément 1) avec un agent complexant capable de complexer l'oxygène plus rapidement que le monoxyde d'azote et 2) avec des cristaux de phtalocyanine de lithium radicalaire, et on mesure le signal RPE desdits cristaux.

La mise en contact du milieu avec l'agent complexant peut être effectuée dans un tube contenant des cristaux de phtalocyanine de lithium radicalaire, muni à sa partie supérieure d'un filtre imprégné d'une solution de l'agent complexant.

Lorsque la mise en contact du milieu avec l'agent complexant et la phtalocyanine de lithium s'effectue en deux étapes successives, on peut déterminer le signal RPE des cristaux de phtalocyanine de lithium à n'importe quel moment.

En revanche, lorsque l'on met en contact le milieu simultanément avec l'agent complexant et les cristaux, il est nécessaire d'effectuer la mesure du signal RPE des cristaux relativement rapidement, c'est-à-dire dans un délai tel que le monoxyde d'azote ne soit pratiquement pas complexé par l'agent complexant.

A titre d'agents complexants susceptibles d'être utilisés, on peut citer les dithionites comme le dithionite de sodium qui complexe très rapidement l'oxygène et complexe également le NO mais avec une cinétique beaucoup plus lente (environ 1h pour diminuer de moitié une teneur en NO de 10⁻⁴ mol/l dans 1ml de solution) alors que la complexation de l'oxygène est quasi-instantanée.

Aussi, en effectuant la mesure du signal RPE rapidement après la mise en contact simultanée d'une solution avec le dithionite et la phtalocyanine de lithium radicalaire, par exemple moins de 30min après cette mise en contact, le NO n'est pratiquement pas complexé et le signal mesuré est représentatif de la concentration en NO.

Dans tous les cas, pour examiner le signal RPE de la phtalocyanine, on peut utiliser un spectromètre à résonance paramagnétique électronique avec des niveaux d'excitation (champ radiofréquence) et de modulation (champ basse fréquence) très faibles pour ne pas saturer le signal et observer la largeur de raie minimum, par exemple avec une puissance d'excitation de quelques µW et une amplitude de modulation de 5 mG.

D'autres caractéristiques et avantages de l'invention apparaîtront mieux à la lecture de la description qui suit, donnée bien entendu à titre illustratif et non limitatif, en référence au dessin annexé sur lequel
- la figure 1 est un diagramme illustrant les variations de la largeur de raie RPE (en mgauss) de deux phtalocyanines de lithium radicalaires en fonction de la concentration en NO d'un milieu liquide,
- la figure 2 représente de façon schématique une conduite destinée à effectuer le contrôle en continu de la teneur en NO d'un milieu liquide,
- la figure 3 représente de façon schématique un récipient permettant le dosage de NO dans un mélange biphasique, et
- la figure 4 représente schématiquement un tube utilisable pour le dosage de NO dans un milieu liquide contenant également de l'oxygène.

On décrit, ci-après, la mesure de la teneur en NO d'une solution par mise en oeuvre du procédé de l'invention en utilisant comme phtalocyanine de lithium la phtalocyanine de formule (I), dans laquelle R¹ à R¹⁶ représentent un atome d'hydrogène, désignée ci-après PcLi.

Cette phtalocyanine de lithium PcLi est sous la forme de cristaux de structure tétragonale appartenant au groupe spatial P4/mcc et elle a été obtenue par le procédé décrit dans FR-A- 2 664 607.

Pour effectuer la mesure, on introduit dans un tube en verre, un échantillon constitué par 0,1 ml d'une solution aqueuse de NO et 3mg de cristaux de phtalocyanine de lithium puis on place le tube dans un spectromètre à résonance paramagnétique électronique, fonctionnant à 280 MHz (100 Gauss), dans des conditions d'observation de la largeur de raie minimum, c'est-à-dire les niveaux d'excitation HF et de modulation BF les plus faibles possibles, et on détermine la largeur de raie RPE de la phtalocyanine de lithium en présence de l'échantillon.

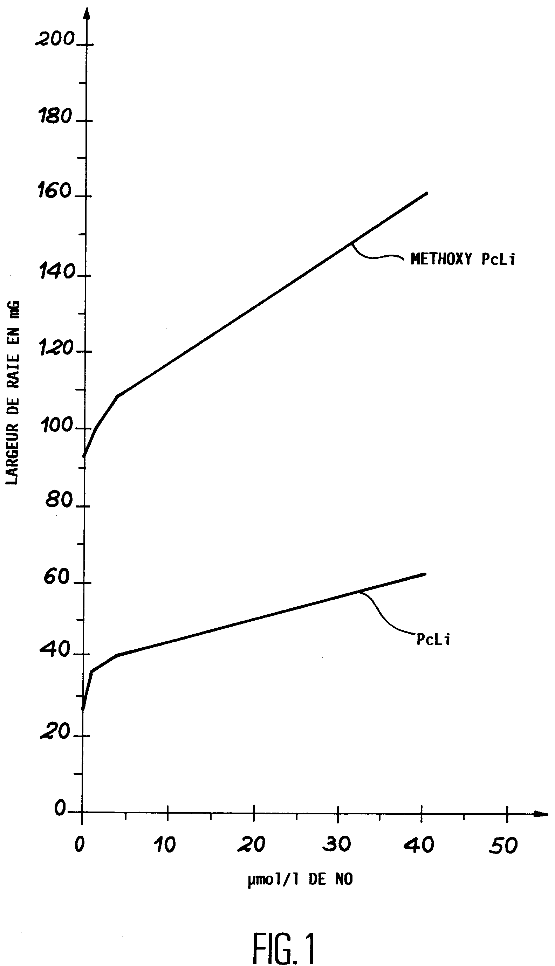

Les résultats obtenus avec différentes solutions aqueuses ayant des concentrations connues en NO sont donnés sur la figure 1 (courbe PcLi) qui représente les variations de la largeur de raie RPE (en mgauss) en fonction de la concentration en NO (en µmol/l) de l'échantillon.

Sur cette figure, on voit que dans la gamme de concentrations en NO allant de 0 à 10µmol/l, on a une évolution très rapide de la largeur de raie RPE, ce qui est particulièrement intéressant pour déterminer des concentrations aussi faibles de NO.

En suivant le même mode opératoire que précédemment, mais en utilisant comme phtalocyanine de lithium radicalaire une phtalocyanine de formule (I) dans laquelle R¹, R⁴, R⁵, R⁸, R⁹, R¹², R¹³, et R¹⁶ représentent un atome d'hydrogène et R², R³, R⁶, R⁷, R¹⁰, R¹¹, R¹⁴ et R¹⁵ représentent le groupe méthoxy, désigné ci-après sous les termes méthoxy PcLi, on obtient la courbe référencée (méthoxy PcLi) sur la figure 1 qui illustre également l'évolution de la largeur de raie (en mgauss) de la méthoxy PcLi en fonction de la concentration (en µmol/l) de NO dans la solution aqueuse.

Ainsi, on voit que l'évolution de la largeur de raie est également très importante dans la gamme de concentrations allant de 0 à 10 µmol/l. La limite de résolution obtenue en utilisant la méthoxy PcLi est de 10⁻⁹mol/l de NO alors qu'elle est de 10⁻⁸mol/l de NO pour PcLi.

Dans les deux cas, cette limite de résolution est très faible, ce qui permet d'effectuer un dosage et un contrôle très précis des teneurs en NO de liquides biologiques.

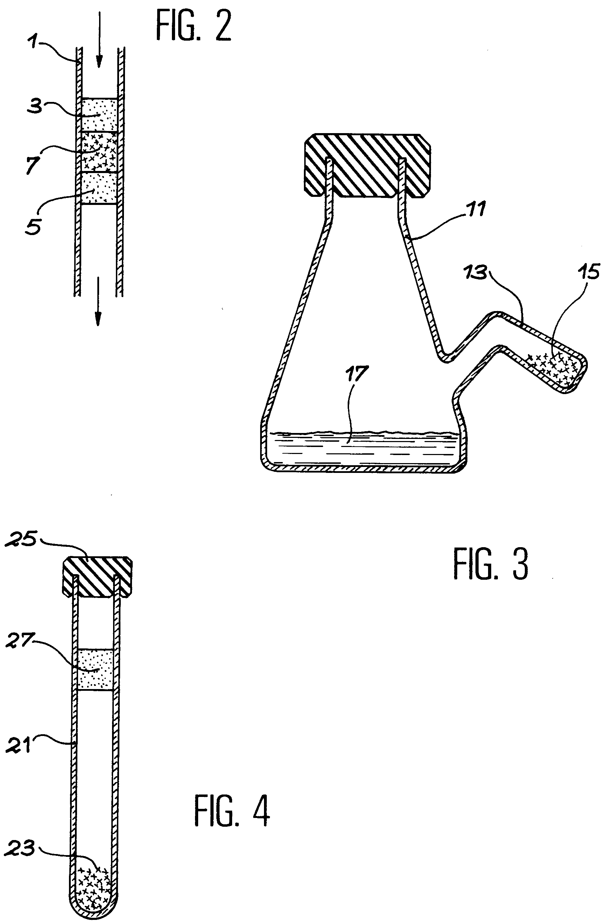

Sur la figure 2, on a représenté un dispositif permettant de réaliser le contrôle en continu de solutions telles que des solutions biologiques titrées.

Sur cette figure, on voit que le dispositif comprend une conduite 1 à l'intérieur de laquelle sont disposées deux pastilles de verre fritté 3 et 5 entre lesquelles est retenue la quantité voulue de cristaux 7 de phtalocyanine de lithium radicalaire. Les pastilles en verre fritté ont une porosité de 2 à 3, qui permet à la solution liquide à contrôler de circuler à l'intérieur de la conduite 1 dans le sens indiqué par les flèches. Cette conduite peut être disposée à l'intérieur d'un spectromètre à résonance paramagnétique électronique et raccordée à ses deux extrémités par des tuyaux souples à une installation dans laquelle on désire contrôler la teneur en NO. Ce contrôle peut être effectué en vérifiant en continu la largeur de raie RPE de PcLi.

Un tel dispositif peut être utilisé en particulier pour le contrôle en flux continu de solutions biologiques titrées, à faible concentration en NO, destinées à une injection chez des patients en soins intensifs.

Sur la figure 3, on a représenté un dispositif convenant au dosage de NO dans des mélanges biphasiques formés d'une solution et d'un gaz.

Ce dispositif comprend un récipient 11 qui communique avec un tube 13 de forme appropriée à l'extrémité duquel sont disposés des cristaux 15 de PcLi. Le tube 13 débouche dans le récipient 11 au-dessus du fond de ce récipient à un niveau tel que, dans la position représentée sur le dessin, le récipient puisse contenir un liquide 17 sans qu'il pénètre dans le tube 13.

Ce récipient convient par exemple à une incubation de cellules in vitro. Ainsi, dans la position représentée, les cristaux placés dans le tube latéral 13 permettent de mesurer le taux du NO gazeux dans un premier temps et ensuite après basculement du récipient dans une autre position, on remplit le tube 13 de liquide et on détermine la concentration en NO du liquide en introduisant dans ces deux cas le tube 13 dans le spectromètre RPE.

Sur la figure 4, on a représenté un autre dispositif utilisable pour la mesure de NO dans une solution contenant également de l'oxygène.

Dans ce cas, le dispositif comprend également un tube 21 contenant des cristaux de PcLi 23 et il peut être fermé par un bouchon septum 25. A la partie supérieure du tube, on a disposé un filtre 27 de papier imbibé d'une solution de dithionite de sodium retenu dans le tube par contrainte mécanique ou par collage tel l'embout filtre des cigarettes par exemple. Pour effectuer la mesure, on introduit l'échantillon à la partie supérieure du tube et par passage sur le filtre 27 imprégné de dithionite de sodium, on élimine toute trace d'oxygène de l'échantillon, ce qui permet de déterminer ensuite uniquement la teneur en NO de cet échantillon qui vient au contact des cristaux de PcLi, par introduction du tube dans un spectromètre RPE.

## Revendications

1. Procédé de détection ou de dosage de monoxyde d'azote NO dans un milieu gazeux et/ou liquide, caractérisé en ce qu'il consiste :
a) à mettre en contact avec ledit milieu des cristaux de phtalocyanine de lithium radicalaire de formule : 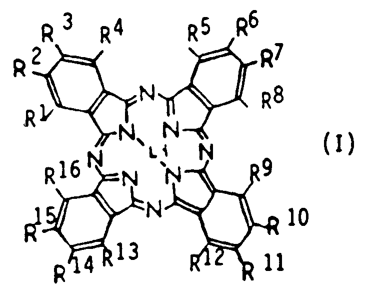 dans laquelle R¹, R⁴, R⁵, R⁸, R⁹, R¹², R¹³ et R¹⁶ représentent un atome d'hydrogène, et R², R³, R⁶, R⁷, R¹⁰, R¹¹, R¹⁴ et R¹⁵ qui peuvent être identiques ou différents, représentent un atome d'hydrogène, le groupe méthyle, ou le groupe méthoxy, et
b) à examiner le signal de résonance paramagnétique électronique (RPE) de la phtalocyanine de lithium radicalaire en contact avec ledit milieu.

2. Procédé selon la revendication 1, caractérisé en ce que l'on contrôle en continu la teneur en monoxyde d'azote d'un milieu gazeux et/ou liquide en faisant circuler en continu ce milieu dans une conduite (1) contenant des cristaux (7) de phtalocyanine de lithium radicalaire et en examinant en continu le signal RPE desdits cristaux.

3. Procédé selon la revendication 2, caractérisé en ce que lesdits cristaux sont retenus dans la conduite entre deux pastilles (3, 5) poreuses perméabless audit milieu.

4. Procédé selon la revendication 1, de dosage des teneurs en NO d'un milieu liquide et d'un milieu gazeux, caractérisé en ce que l'on introduit lesdits milieux dans un récipient (11) fermé comportant un tube latéral (13) étanche à l'intérieur duquel sont retenus des cristaux (15) de phtalocyanine de lithium radicalaire, ledit tube étant en communication avec le récipient et débouchant dans celui-ci à un niveau tel que, dans une première position du récipient, ce dernier contienne le milieu liquide (17) sans qu'il vienne au contact desdits cristaux, on effectue dans cette première position une première mesure du signal RPE des cristaux qui sont en contact uniquement avec le milieu gazeux, puis on amène le récipient dans une seconde position dans laquelle le milieu liquide pénètre dans le tube latéral pour être en contact avec lesdits cristaux et on effectue une seconde mesure de signal RPE desdits cristaux qui sont en contact uniquement avec le milieu liquide.

5. Procédé selon la revendication 1, de dosage ou de détection du monoxyde d'azote dans un milieu contenant de l'oxygène, caractérisé en ce que l'on met ledit milieu en contact simultanément ou successivement 1) avec un agent complexant capable de complexer l'oxygène beaucoup plus rapidement que le monoxyde d'azote, et 2) avec des cristaux de phtalocyanine de lithium radicalaire, et on mesure le signal RPE desdits cristaux.

6. Procédé selon la revendication 5, caractérisé en ce que l'agent complexant est le dithionite de sodium.

7. Procédé selon l'une quelconque des revendications 5 et 6, caractérisé en ce que la mise en contact s'effectue dans un tube (21)contenant des cristaux de phtalocyanine de lithium radicalaire, muni à sa partie supérieure d'un filtre (27) imprégné d'une solution de l'agent complexant.

8. Procédé selon l'une quelconque des revendications 1 à 7, caractérisé en ce que la phtalocyanine de lithium radicalaire répond à la formule (I) avec R¹ à R¹⁶ représentant un atome d'hydrogène.

9. Procédé selon l'une quelconque des revendications 1 à 7, caractérisé en ce que la phtalocyanine de lithium radicalaire répond à la formule I avec R¹, R⁴, R⁵, R⁸, R⁹, R¹², R¹³ et R¹⁶ représentant un atome d'hydrogène et R², R³, R⁶, R⁷, R¹⁰, R¹¹, R¹⁴ et R¹⁵ représentant le groupe méthoxy.
